(19) Europäisches Patentamt
European Patent Office
Office européen des brevets

(11) Publication number: 0 131 811
B1

(12) # EUROPEAN PATENT SPECIFICATION

(45) Date of publication of patent specification: 19.08.87

(51) Int. Cl.$^4$: C 07 D 205/08, C 07 D 499/00

(21) Application number: 84107463.6

(22) Date of filing: 28.06.84

(54) **Process for the preparation of azetidinones.**

(30) Priority: 14.07.83 US 513887

(43) Date of publication of application:
23.01.85 Bulletin 85/04

(45) Publication of the grant of the patent:
19.08.87 Bulletin 87/34

(84) Designated Contracting States:
AT BE CH DE FR GB IT LI NL SE

(56) References cited:
EP-A-0 013 662
EP-A-0 042 026
EP-A-0 082 113
US-A-4 301 074

(73) Proprietor: SCHERING CORPORATION
2000 Galloping Hill Road
Kenilworth, New Jersey 07033 (US)

(72) Inventor: Steinman, Martin
46 Glendale Avenue
Livingston New Jersey 07039 (US)
Inventor: Wong, Yee Shing
13 Brighton Avenue
Belleville New Jersey 07109 (US)

(74) Representative: Ritter, Stephen David et al
Mathys & Squire 10 Fleet Street
London EC4Y 1AY (GB)

EP 0 131 811 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to an improved process for the preparation of azetidinones from penicillinates. The azetidinones are *inter alia* useful intermediates in the preparation of other compounds, for example penems.

Penems themselves are well known compounds having antibacterial activity. For instance the compound sodium (5R,6S,8R)-6-(1-hydroxyethyl)-2-ethylthiopenem-2-carboxylate is active against *Staphylococcus* 76070103 at a test level of less than 0.06 µg/ml and against E. coli at a test level of 0.5 µg/ml.

Various routes to a wide variety of penem compounds have been described, for instance in European Patent Applications, publication Nos. 0 003960, 0 013662, 0 035188 and 0 110280. Such routes generally proceed from 6-aminopenicillanic acid (6-APA) by a plurality of steps, the initial ones of which comprise esterification of the carboxyl group by e.g. a methyl group and, if desired, conversion of the 6-position substituent to another substituent e.g. 1-hydroxyethyl, followed by cleavage of the thiazolidine ring of the penicillinate, so as to form a 4-acyloxy or 4-aroyloxy azetidinone intermediate.

One widely used method of cleaving the thiazolidine ring (e.g. in U.S. 4301074) utilizes mercuric acetate as the cleaving agent. This method has the disadvantage of requiring the disposal or recycling of the mercury.

Suharto et al., Tetrahedron Letters No. 42, 4059—62 (1978) disclose a method in which the penicillinate is converted to the 1-oxide which is then cleaved with a carboxylic acid in the presence of trimethylphosphite. This method has the disadvantage of requiring the use of the 1-oxide penam.

The present invention is aimed at providing a simple procedure capable of preparing azetidinone compounds from penicillinates in high yields.

According to the present invention a compound of the formula I

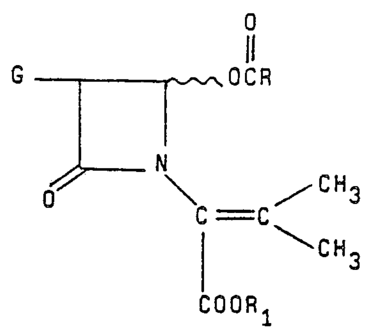

I

in which G is hydroxyloweralkyl, in which the hydroxy function is protected by a removable hydroxy-protecting group, $R_1$ is loweralkyl, a carboxy protecting group or a metabolisable ester group, R is an organic group inert to the reagents specified in steps (a) and (b) below, and the wavy line indicates a mixture of compounds having the R and S chemical configurations at the 4 carbon atom, is prepared by

a) treating a penam of formula II with a nitrate of formula III in an inert organic solvent, e.g. a halogenated hydrocarbon, such as 1,2-dichloroethane, so as to produce a compound of formula IV according to the reaction scheme

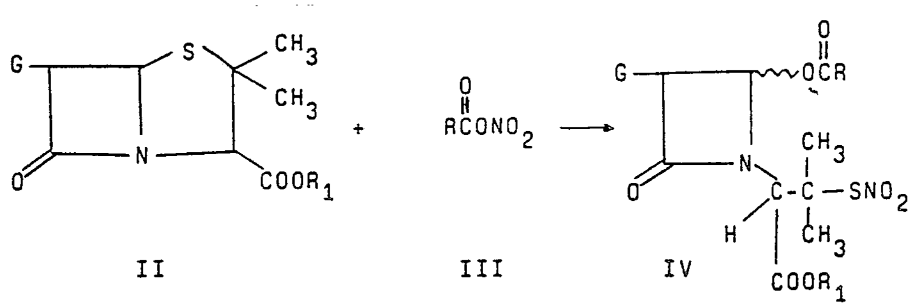

II III IV

in which G, R and $R_1$ and the wavy line are as defined above, followed by

b) converting the compound of formula IV to a compound of formula I as defined above by treatment thereof with an organic base.

R is preferably alkyl, cycloalkyl or substituted or unsubstituted aryl and is most preferably loweralkyl, phenyl or phenyl substituted with up to three substituents independently chosen from nitro, halo and loweralkyl.

G is preferably 1-protected-hydroxyethyl.

A preferred carboxy protecting group is allyl.

Preferably the starting penam compound II has the structure and stereoconfiguration indicated in the drawing below:

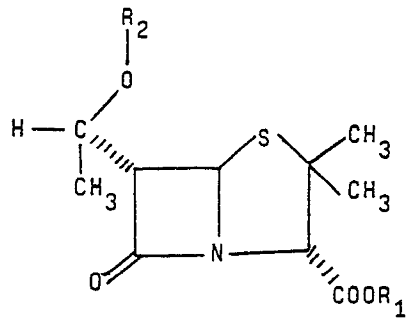

II'

in which $R_2$ is a removable hydroxyprotecting group. The corresponding compound of formula IV obtained in step a) is as shown below:

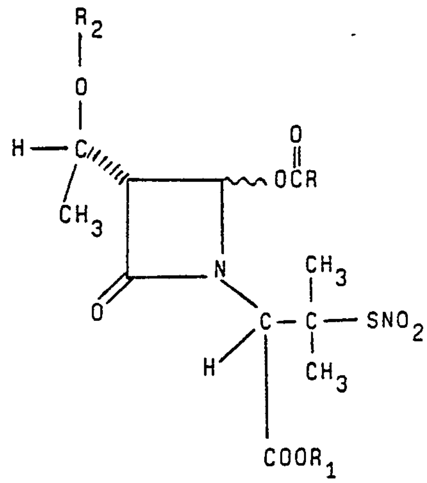

IV'

and the final corresponding compound of formula I has the structure and stereoconfiguration of formula I' as shown below:

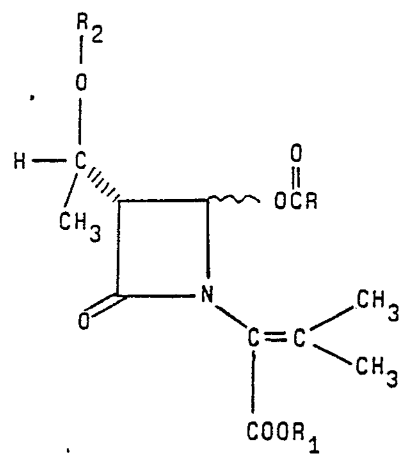

I'

The reaction of step a) should be anhydrous and is generally carried out under cool conditions, i.e. usually below room temperature, e.g. about 5°C to about 20°C. The reaction generally takes up to 12 hours to complete.

Suitable organic bases for use in converting compounds IV to compounds I are trialkylamines; the preferred organic base is trialkylamine.

The conversion of the intermediate compound IV to compound I is preferably carried out without isolating it from the reaction mixture from step a) and is generally completed in 4 to 8 hours. As a general rule the pH for the conversion will be greater than 10.

The product compound of formula I is recovered in high yield e.g. equal to or greater than 80% based on the starting penam, possibly ⩾85%, for example a yield of 87% as achieved in Example 1 herein.

As used herein and throughout the specification and claims

"alkyl" means a straight or branched chain alkyl group containing 1 to 12 carbon atoms and "loweralkyl" such group containing 1 to 6 carbon atoms e.g. methyl, ethyl, propyl, isopropyl, butyl, pentyl and the like,

"aryl" means a monovalent aromatic hydrocarbon group such as phenyl and naphthyl,

"cycloalkyl" means a cycloaliphatic group of 3 to 12 carbon atoms preferably 3 to 7 carbon atoms, such as cyclopropyl, cyclobutyl, cyclopentyl, cyclohexyl and cycloheptyl,

"halo" means halogen such as chlorine or bromine,

"metabolisable ester group" means a physiologically cleavable ester group i.e. groups forming those esters known in the penicillin, cephalosporin and penem arts to be easily cleaved within the body to the parent acid. Examples of such ester groups are indanyl, phthalidyl, methoxymethyl, glycyloxymethyl, phenylglycyloxymethyl, thienylglycyloxymethyl, acetoxymethyl and pivaloyloxymethyl. The esters are prepared by conventional procedures for making esters of penicillins.

The term "removable hydroxy-protecting group", means any such group conventionally used for this purpose with the only requirement being compatibility with the hydroxy substituent on the molecules derived by further conversion of the formula I compounds e.g. penems, and removability utilizing elemental zinc or any other conventional agent for this purpose which will not adversely affect the structure of such molecules. Preferred are those groups which have an oxycarbonyl moiety, e.g. trichloroethoxycarbonyl and paranitrobenzyloxycarbonyl, and trimethylsilyl.

The acyl or aroyl nitrate reactant I can be prepared by conventional means, i.e. reaction of the corresponding acid anhydride with nitric acid. The preferred nitrate is acetylnitrate.

The following Examples illustrate the invention:

## Preparation 1

Acetyl Nitrate

Cool 50 ml of 1,2-dichloroethane and 50 ml of acetic anhydride (0.5 mole) at 10°C under nitrogen using a drying tube. Add 0.5 ml of concentrated sulfuric acid followed by 8 ml of 90% nitric acid (0.2 mole) dropwise to the mixture with stirring at 10°C to 15°C for 2.5 hours. The product, acetyl nitrate, is used without isolation in the succeeding reaction with a penicillinate ester as in Example I below.

## Preparation 2

Propionyl Nitrate

Add to 6.5 ml of propionic anhydride (0.05 mole) in 5 ml of 1,2-dichloroethane under nitrogen using a drying tube, 2 drops of concentrated sulfuric acid and then add 0.8 ml of 90% nitric acid (0.02 mole) dropwise with stirring at 20°C (inside temperature) for 3 hours. The product, propionyl nitrate, is used without isolation in the succeeding reaction with a penicillinate ester as in Example 2 below.

Following the procedures of Preparation 1 and 2, aroyl nitrates such as benzoyl nitrate are prepared using the appropriate acid anhydride.

## Example 1

(3S,5R)-1-[2-methyl-1-methoxycarbonylprop-1-enyl]-3-(1-(2,2,2)trichloroethoxy-carbonyloxyethyl)-4-acetoxyazetidin-2-one

Add 43.5 g of methyl (5R,6S,8R)-6-(1-(2,2,2)trichloroethoxycarbonyloxyethyl)penicillinate [prepared as described in DiNinno et al., J. Org. Chem. *42*, 2960 (1977)] in 50 ml of 1-2-dichloroethane slowly at 10°C to 15°C to the final reaction mixture of Preparation 1. After addition is completed, stir the mixture at 15°C for 3 hours and pour into 200 ml of ice-water and continue stirring overnight at room temperature. Separate the organic layer and wash with water (200 ml). Add triethylamine (50 ml) slowly to the cold organic mixture, with stirring, over a period of 4 hours (final pH 10), and then wash with water (3 × 100 ml). Remove the solvent under vacuum to dryness. Chromatograph the residue on silica gel (hexane/methylene chloride = 1:1) to obtain the title compound as an oil in 87% yield based on the starting penicillanate.

## Example 2

(3S,5R)-1-[2-methyl-1-methoxycarbonylprop-1-enyl]-3-(1-(2,2,2)trichloroethoxycarbonyloxyethyl)-4-propionoxyazetidin-2-one

Add 4.3 g of methyl(5R,6S,8R)-6-(1-(2,2,2)trichloroethoxycarbonyloxyethyl)penicillinate in 5 ml of 1,2-dichloroethane slowly at 15°C to the final reaction mixture of Preparation 2. After addition is completed, stir the mixture at 20°C for 4 hours, and pour into 20 ml of ice-water and continue stirring overnight at room temperature. Separate the organic layer and wash with water (20 ml). Add triethylamine (5 ml) slowly to the cold organic mixture, with stirring, over a period of 4 hours (final pH 10), and then wash with water (3 × 10 ml). Treat the organic layer with 30 ml of 5.25% sodium hypochlorite (Clorox). Separate the organic layer, stir with 5 ml triethylamine for 3 hours and then wash with water (3 × 20 ml). Dry the organic layer over anhydrous sodium sulfate and filter through silica gel. Remove the solvent to dryness to obtain the title compound as an oil in 80% yield based on the starting penicillanate.

The compounds of formula I in which R is phenyl or substituted phenyl are prepared in high yields by the methods of Examples 1 and 2, utilizing the appropriate nitrate reagent.

The preparation of antibacterially active compounds from the compounds of formula I can, as mentioned above, be carried out by methods well known in the art, as briefly described below for example;

REACTION SCHEME

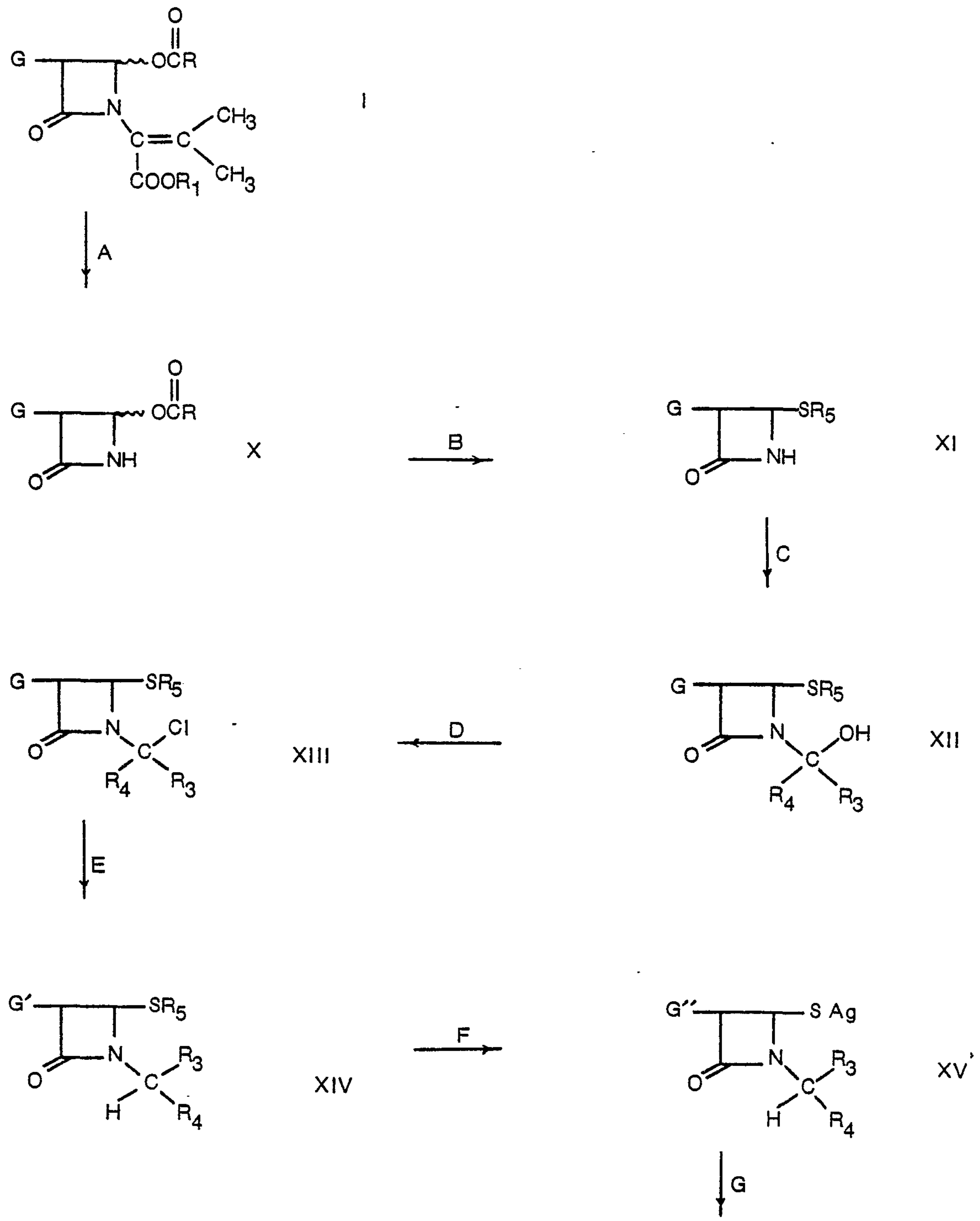


I

X

XI

XII

XIII

XIV

XV'

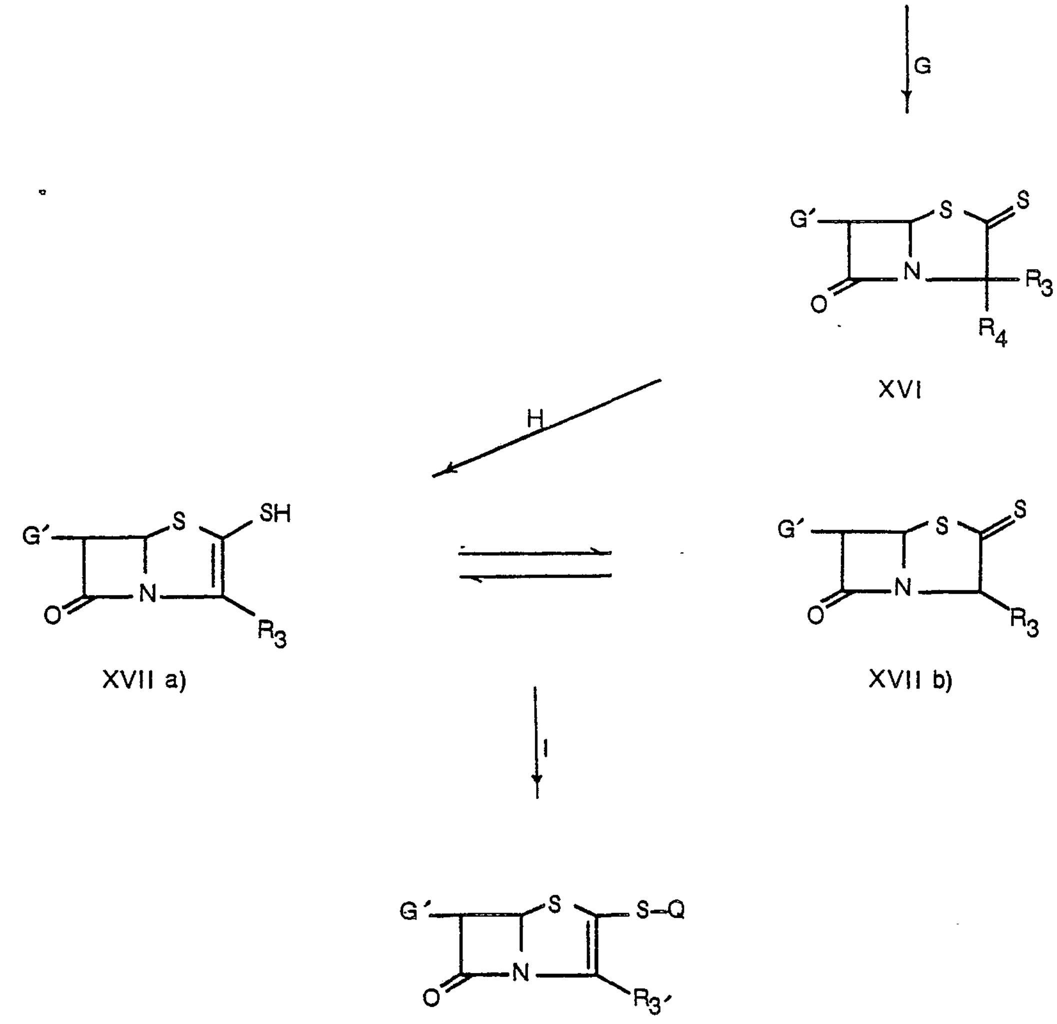


XVI

XVII a)

XVII b)

XVIII

Step A can be carried out by treating compound I in a mixture of acetone and water with potassium permanganate. See also U.S. Patent 4301074. G represents 1-(2,2,2)-trichloroethoxycarbonyloxyethyl-.

Steps B to I are described in European Patent Application publication No. 0 110280. That is to say Step B involves replacing the acyloxy or aroyloxy group with the group triphenylmethylthio represented as —$SR_5$. This can be carried out by reacting compound X with triphenylmethylthiol in acetonitrile in the presence of caesium carbonate, and compound XI having stereoconfiguration 3S,4R isolated by chromatography in conventional manner.

Step C is carried out by reacting compound XI with allyltrimethylsilylketomalonate in dimethylformanide in the presence of a small amount of pyridine and triethylamine at room temperature followed by solvent removal. $R_3$ represents allyloxycarbonyl and $R_4$ represents trimethylsilylethoxy carbonyl.

Step D is carried out by treating compound XII with thionyl chloride, in solution in methylene chloride in the presence of pyridine and some calcium carbonate at about 0°C to 5°C. Compound XIII is isolated by chromatography in conventional manner.

In Step E compound XIII is subjected to simultaneous dechlorination and deprotection of group G by treatment via zinc/acetic acid at 0°C. G′ represents 1-hydroxyethyl.

The resulting compound XIV is treated in Step F with silver nitrate in solution in methanol and in the presence of a small amount of pyridine under a nitrogen atmosphere. The reaction mixture is worked up in conventional manner and the isolated compound is dissolved in methylene chloride and treated with bis-trimethylsilylacetamide at room temperature to reprotect the hydroxy group of grouping G′ (the protecting group being trimethylsilyl), giving compound XV.

In Step G reaction mixture from Step F is treated with thiocarbonyl diimidazole at room temperature and the resulting penam product of stereoconfiguration 5R,6S,8R is isolated by chromatography; this is then treated in aqueous tetrahydrofuran with acetic acid to deprotect group G″. The solution is worked up in conventional manner to give compound XVI.

In Step H a solution of compound XVI in tetrahydrofuran is treated with tetrabutylammonium fluoride

giving the tautomeric compound [(XVIIa),(XVIIb)].

The final penem compounds XVIII can be prepared from tautomeric compound [(XVIIa),(XVIIb)] by known reactions. For instance reaction of the tautomer with ethyl iodide gives a final product in which Q is ethyl. The group $R_3$ can be deprotected as described in European Patent Application publication No. 0 013663. That is, the allylic group can be removed by utilizing tetrahydrofuran solvent with sodium 2-ethylhexanoate and a mixture of a palladium compound and triphenylphosphine as catalyst to give the penem sodium salt ($R_3'$ being —COONa).

**Claims**

1. A process for preparing a compound of formula I

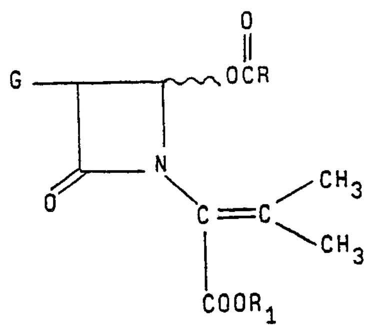

I

in which

G is protected-hydroxy $C_1$—$C_6$ alkyl,

$R_1$ is $C_1$—$C_6$ alkyl, a carboxy protecting group or a metabolizable ester group,

R is an organic group inert to the reagents specified in steps (a) and (b) below and

the wavy line indicates a mixture of compounds having the R and S stereo-chemical configuration at the 4 carbon atom, characterised in that,

a) a penam of formula II is treated with a nitrate of formula III in an inert organic solvent, so as to produce a compound of formula IV, according to the reaction scheme

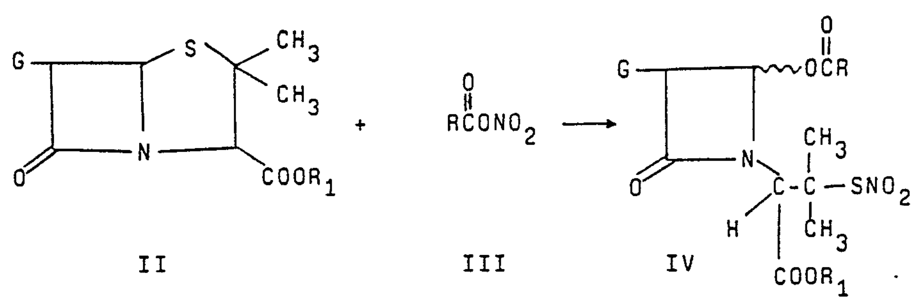

II III IV

in which G, R and $R_1$ are as defined above, and

b) the resulting compound of formula IV is converted to the desired compound of formula I by treatment with an organic base.

2. A process as defined in claim 1, characterised by using a compound II having the structure and stereochemical configuration represented by formula II'

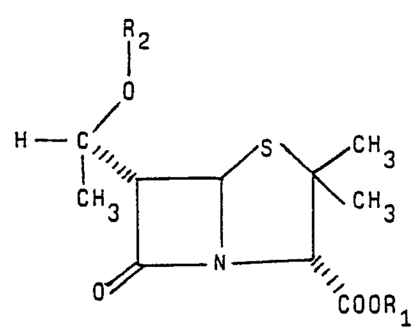

II'

in which $R_1$ is $C_1$—$C_6$ alkyl, allyl or a metabolisable ester group and $R_2$ is a hydroxy-protecting group,

whereby there is obtained in step a) a compound having the structure and stereoconfiguration as shown in formula II'

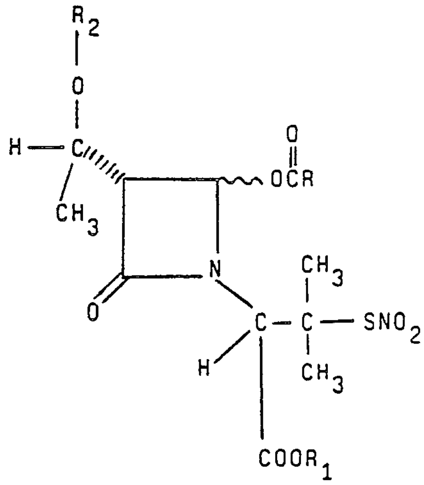

IV'

and there is obtained in step b) a compound having the structure and stereoconfiguration as shown in formula I'

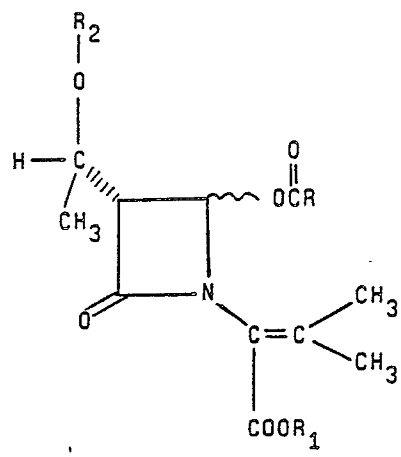

I'

wherein the wavy line is as defined in claim 1.

3. A process as defined in claim 1 or 2 characterised in that the treatment of penam II or II' with the acyl or aroyl nitrate is carried out under cool conditions, preferably about 5°C. to about 20°C.

4. A process as defined in any one of the preceding claims, characterised by using triethylamine as the organic base.

5. A process as defined in any one of claims 1 to 4, characterised in that acetyl nitrate is used as the compound of formula III.

6. A process as defined in any one of the preceding claims, characterised in that (5R,6S,8R) methyl-6-(1-(2,2,2)-trichloroethoxycarbonyloxy-ethyl) penicillinate is used as the compound of formula II.

7. A compound of formula IV or IV' as defined in claim 1 or 2, possibly in solution in an organic solvent.

**Patentansprüche**

1. Verfahren zur Herstellung einer Verbindung der Formel I

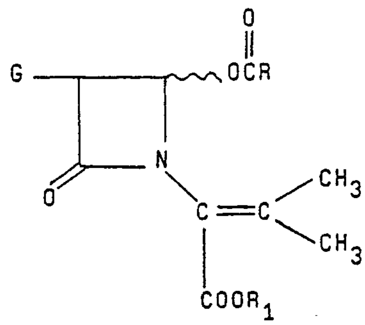

I

in welcher G geschütztes Hydroxy-$C_1$—$C_6$-Alkyl, $R_1$ $C_1$—$C_6$-Alkyl, eine Carboxyschutzgruppe oder eine metabolisierbare Estergruppe und R eine gegen die in den folgenden Stufen a) und b) angeführten Reaktionsteilnehmer inerte organische Gruppe bedeutet und die Wellenlinie eine Mischung von Verbindungen mit R- und S-stereochemischer Konfiguration am 4-Kohlenstoffatom bezeichnet, dadurch gekennzeichnet, daß

a) ein Penam der Formel II mit einem Nitrat der Formel III in einem inerten organischen Lösungsmittel behandelt wird, um eine Verbindung der Formel IV gemäß dem Reaktionsschema

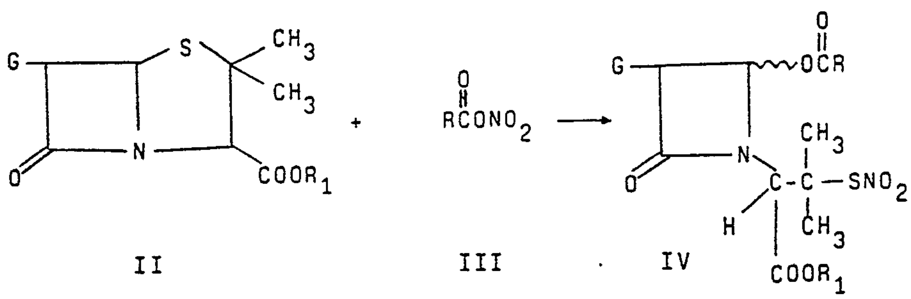

II III IV

in welchem G, R und $R_1$ obige Bedeutung haben, herzustellen und

b) die entstandene Verbindung der Formel IV in die gewünschte Verbindung der Formel I durch Behandlung mit einer organischen Base übergeführt wird.

2. Verfahren nach Anspruch 1, gekennzeichnet durch Verwendung einer Verbindung II mit der in Formel II' wiedergegebenen Struktur und stereochemischen Konfiguration

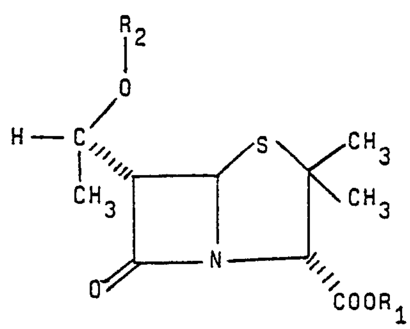

II'

in welcher $R_1$ $C_1$—$C_6$-Alkyl, Allyl oder eine metabolisierbare Estergruppe und $R_2$ eine Hydroxyschutzgruppe bedeutet, wodurch in Stufe a) eine Verbindung mit der in Formel II' gezeigten Struktur und Stereokonfiguration erhalten wird.

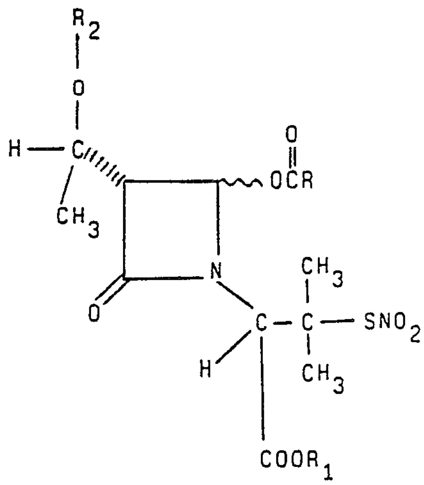

IV'

und in Stufe b) eine Verbindung mit der in Formel I' gezeigten Struktur und Stereokonfiguration erhalten wird.

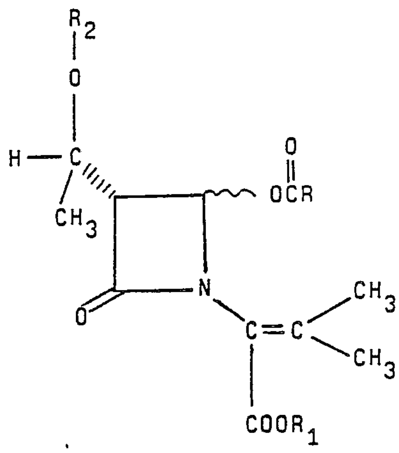

I'

worin die Wellenlinie wie in Anspruch 1 definiert ist.

3. Verfahren nach Anspruch 1 oder 2, dadurch gekennzeichnet, daß die Behandlung des Penams II oder II' mit dem Acyl- oder Aroylnitrat unter Kühlbedingungen, vorzugsweise bei etwa 5°C bis etwa 20°C, durchgeführt wird.

4. Verfahren nach einem der vorhergehenden Ansprüche, gekennzeichnet durch die Verwendung von Triethylamin als organische Base.

5. Verfahren nach einem der Ansprüche 1 bis 4, dadurch gekennzeichnet, daß als Verbindung der Formel III Acetylnitrat verwendet wird.

6. Verfahren nach einem der vorhergehenden Ansprüche, dadurch gekennzeichnet, daß als Verbindung der Formel II (5R,6S,8R)-Methyl-6-(1-(2,2,2)-trichlorethoxycarbonyloxy-ethyl)-penicillinat verwendet wird.

7. Verbindung der Formel IV oder IV' gemäß Anspruch 1 oder 2, gegebenenfalls in Lösung in einem organischen Lösungsmittel.

**Revendications**

1. Procédé de préparation d'un composé de formule I

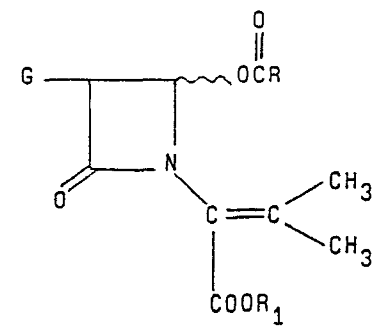

I

dans laquelle

G est un alcoyle $C_1$—$C_6$ hydroxy-protégé.

$R_1$ est alcoyle $C_1$—$C_6$, un groupe carboxy-protecteur ou un groupe ester métabolisable,

R est un groupe organique inerte aux réactifs spécifié aux étapes a) et b) ci-dessous et

la ligne ondulée indique un mélange de composés ayant les configurations stéréochimiques R et S à l'atome de carbone 4, caractérisé en ce que

a) un pénam de formule II est traité avec un nitrate de formule III dans un solvant organique inerte afin de produire un composé de formule IV, selon le schéma réactionnel

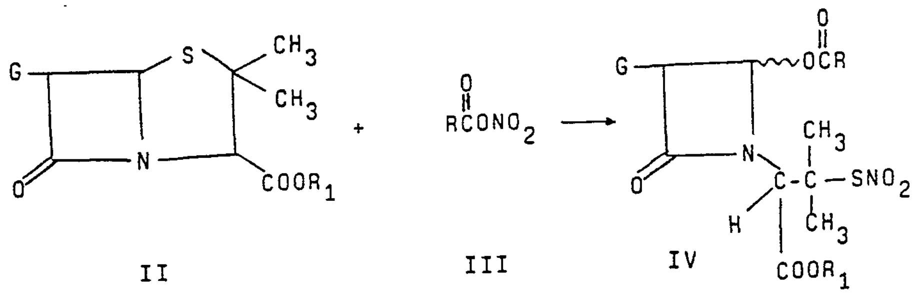

II III IV

où G, R et $R_1$ sont tels que définis ci-dessus, et

b) le composé résultant de formule IV est converti en composé souhaité de formule I par traitement avec une base organique.

2. Procédé selon la revendication 1, caractérisé en ce qu'on utilise un composé II ayant la structure et la configuration stéréochimique représentées par la formule II'

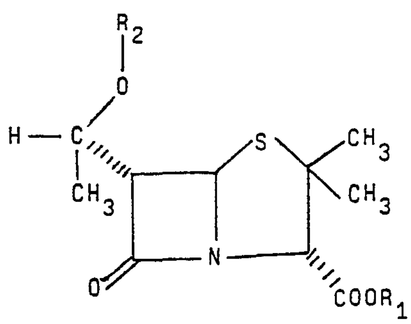

II'

où $R_1$ est alcoyle $C_1$—$C_6$, allyle ou un groupe ester métabolisable et $R_2$ est un groupe hydroxy-protecteur, on obtient ainsi à l'étape a) un composé ayant la structure et la stéréoconfiguration montrées à la formula II'

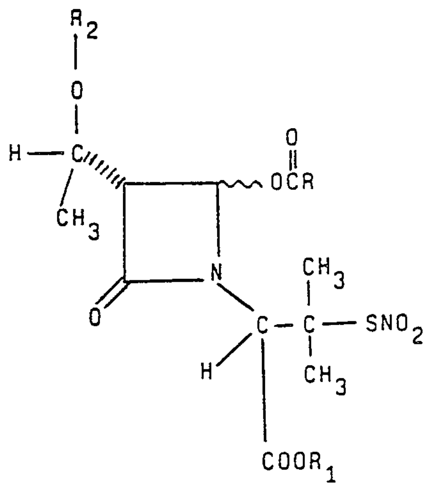

IV'

et on obtient à l'étape b) un composé ayant la structure et la stéréoconfiguration montrées à la formule I'

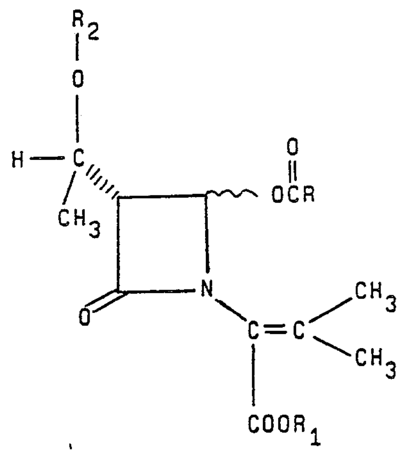

I'

où la ligne ondulée est telle que définie à la revendication 1.

3. Procédé selon la revendication 1 ou 2, caractérisé en ce que le traitement du pénam II ou II' avec le nitrate d'acyle ou d'aroyle est effectué en conditions fraîches, de préférence environ 5°C à environ 20°C.

4. Procédé selon l'une quelconque des revendications précédentes, caractérisé en ce que l'on utilise la triéthylamine comme base organique.

5. Procédé selon l'une quelconque des revendications 1 à 4, caractérisé en ce que l'on utilise du nitrate d'acétyle comme composé de formule III.

6. Procédé selon l'une quelconque des revendications précédentes, caractérisé en ce que l'on utilise, comme composé de formule II, le (5R,6S,8R) méthyl-6-(1-(2,2,2)-trichloroéthoxycarbonyloxyéthyl)pénicillinate.

7. Composé de formule IV ou IV' selon la revendication 1 ou 2, éventuellement en solution dans un solvant organique.